# EUROPEAN PATENT SPECIFICATION

(11) **EP 4 302 756 B1**
(45) Date of publication and mention of the grant of the patent: **05.11.2025**
(21) Application number: 23183529.9
(22) Date of filing: 05.07.2023
(51) Int. Cl.: A61K 31/05, A61K 31/7048, A61K 45/06, A61P 35/00

(54) **PHARMACEUTICAL COMPOSITION COMPRISING CANNABIDIOL AND ETOPOSIDE FOR USE IN THE TREATMENT OF LUNG CANCER**
PHARMAZEUTISCHE ZUSAMMENSETZUNG ENTHALTEND CANNABIDIOL UND ETOPOSID ZUR BEHANDLUNG VON LUNGENKREBS
COMPOSITION PHARMACEUTIQUE COMPRENANT DU CANNABIDIOL ET DE L'ÉTOPOSIDE POUR SON UTILISATION DANS LE TRAITEMENT DU CANCER DU POUMON

(30) Priority: 05.07.2022 KR 20220082356; 12.01.2023 KR 20230004922
(43) Date of publication of application: 10.01.2024
(73) Proprietor: Neocannbio Co., Ltd., Seoul 02792 (KR)
(72) Inventor: HAM, Jung Yeob, 25400 GANGNEUNG-SI (KR); KIM, Jeong Kook, 01000 SEOUL (KR); SONG, Jung Ho, 01000 SEOUL (KR); JEONG, Kyu Hyuk, 15523 SUWON-SI (KR)
(74) Representative: Comoglio, Elena

(56) References cited:
- WO-A1-2017/072773
- WO-A1-2020/194237
- SELTZER EMILY S. ET AL: "Cannabidiol (CBD) as a Promising Anti-Cancer Drug", CANCERS, vol. 12, no. 11, 30 October 2020 (2020-10-30), pages 3203, XP055914780, DOI: 10.3390/cancers12113203
- REZONJA RENATA ET AL: "Oral treatment with etoposide in small cell lung cancer - dilemmas and solutions", RADIOLOGY AND ONCOLOGY, vol. 47, no. 1, 1 January 2013 (2013-01-01), SI, pages 1 - 13, XP093094891, ISSN: 1318-2099, DOI: 10.2478/raon-2013-0008
- KANDER ET AL: "Cannabis for the Treatment of Cancer: The Anticancer Activity of Phytocannabinoids and Endocannabinoids", DRUGPOLICY.ORG, 20 April 2016 (2016-04-20), XP055659464, Retrieved from the Internet <URL:http://www.drugpolicy.org/sites/default/files/Cannabis_and_Cancer_ARblog_081115.pdf> [retrieved on 20200120]
- WHYNOT ERIN G. ET AL: "Anticancer properties of cannabidiol and [Delta]9-tetrahydrocannabinol and synergistic effects with gemcitabine and cisplatin in bladder cancer cell lines", JOURNAL OF CANNABIS RESEARCH, vol. 5, no. 1, 4 February 2023 (2023-02-04), XP093094648, DOI: 10.1186/s42238-023-00174-z
- KURO-O M: "Molecular Mechanisms Underlying Accelerated Aging by Defects in the FGF23-Klotho System", INTERNATIONAL JOURNAL OF NEPHROLOGY, vol. 2018, 21 May 2018 (2018-05-21), pages 1 - 6, XP093258967, ISSN: 2090-214X, DOI: 10.1155/2018/9679841

## Description

### BACKGROUND

### 1. Field

This disclosure relates to pharmaceutical compositions for preventing or treating lung cancer including cannabidiol (CBD) and etoposide as active ingredients, for use in preventing or treating lung cancer.

### 2. Description of the Related Art

*Cannabis (Cannabis sativa* L.) is an annual plant belonging to the genus *Cannabis* in the family Cannabaceae, which has been widely cultivated in temperate and tropical areas, mainly in Central Asia, for 12,000 years, and includes wild-type *Cannabis,* and collectively refers to *Cannabis chemovars,* which contain different kinds of cannabinoid compounds known as medical/pharmaceutical ingredients, and variants thereof, *Cannabis sativa* subspecies *sativa* including variants var. *indica* and var. *kafiristanica, Cannabis sativa* subspecies *indica, Cannabis sativa* subspecies *ruderalis,* and plants which are the result of genetic-crosses, self-crosses, or hybridized plant thereof.

According to Korean and Chinese traditional medical records, mazain or hwamain, which is a peeled seed of *Cannabis,* has been used for constipation, diabetes, pain disorders, menstrual irregularities, skin diseases, dysentery, etc., and *Cannabis* weed which is a *Cannabis* leaf has been used for anthelmintic, hair protection, asthma, analgesic, anesthetic, diuretic purposes, etc. In addition, *Cannabis* root has been used to treat difficult deliveries and to relieve blood stasis, *Cannabis* skin has been used for bruises, irritating rashes and distending pain, *Cannabis* flower has been used for paralysis, itching, etc., and *Cannabis* flower neck has been used for difficult deliveries, constipation, gout, insanity, insomnia, etc., there are records that each part of *Cannabis* were appropriately used according to the symptoms.

There are approximately 400 compounds in *Cannabis,* the majority of which are cannabinoids, terpenes, and phenolic compounds, with more than 90 cannabinoids of medical/pharmaceutical importance, many of which are found only in *Cannabis.* Among the cannabinoids of *Cannabis,* a psychotropic ingredient is Δ9-tetrahydrocannabinol (Δ9-THC), and cannabidiol (CBD), which is a non-psychotropic ingredient, are known to exhibit physiologically active effects through various receptors in the human body, including adrenergic receptors and cannabinoid receptors.

Meanwhile, according to an analysis by the Statistical Office of Korea, cancer is the number one cause of death in South Korea, accounting for 31.7 % of all deaths in men and 22.5 % in women. There are several ways to treat cancer, including surgery, radiation therapy, and chemotherapy, but these treatments have side effects or are limited in their effectiveness depending on the severity of the cancer. In particular, since most anti-cancer agents work by stopping the cell cycle of dividing cells and causing them to die, they also attack cells that are dividing normally, causing side effects such as hair loss, loss of appetite, and decreased immunity due to low white blood cells. Therefore, there is a need for techniques to maximize the therapeutic effect of anti-cancer agents while using low concentrations.

WO 2017/072773 A1 discloses combinations of cannabidiol (CBD) and the anti-cancer agent tamoxifen for use in an *in vitro* model of the treatment of cancer, including lung cancer. Combinations of CBD and etoposide are not disclosed.

WO 2020/194237 A1 discloses combinations of CBD and an anti-cancer drug, preferably for the treatment of lung cancer. Etoposide is disclosed as one of a number of possible chemotherapeutic agents, but no enabling disclosure is provided in connection with the combination of CBD and etoposide in the treatment of lung cancer.

Cancers 2020, 12(11), 3203 and Radiol. Oncol. 2013, 47(1), 1 disclose the use of CBD or etoposide, respectively, in the treatment of small cell lung cancer; however, such references do not disclose the combination of CBD and etoposide.

Based on this background, the disclosure was completed by demonstrating the significantly enhanced anti-cancer efficacy in response to the combined treatment of etoposide and CBD.

### SUMMARY

Provided is a pharmaceutical composition for use in preventing or treating lung cancer, including cannabidiol (CBD) and an anti-cancer agent as an active ingredient, wherein the anti-cancer agent is etoposide.

Additional aspects will be set forth in part in the description which follows and, in part, will be apparent from the description, or may be learned by practice of the presented embodiments of the disclosure.

An aspect provides pharmaceutical compositions for use in preventing or treating lung cancer includes CBD and an anti-cancer agent as active ingredients, wherein the anti-cancer agent is etoposide.

As used herein, the term "cannabidiol (CBD)" refers to one of the main compounds in *Cannabis,* represented by the following Formula 1 (IUPAC name: 2-[(1R,6R)-3-methyl-6-prop-1-en-2-ylcyclohex-2-en-1-yl]-5-pentylbenzene-1,3-diol), which is often compared to tetrahydrocannabinol (THC), a substance that causes hallucinations. In Korea, CBD is designated as a narcotic drug, so not much research is being conducted, but in foreign countries, CBD is used for medical purposes to relieve symptoms such as pain, memory disorders, and anxiety, and CBD is known to suppress excessive oil production, so research on cosmetic ingredients for acne skin is also active. There are also several reports suggesting that CBD may be effective in epilepsy syndromes that cause seizures by participating in the excitatory-inhibitory equilibrium of neural activity.

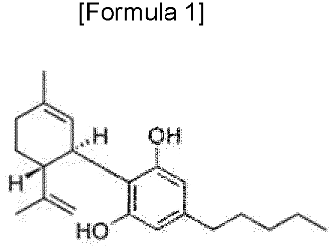

The CBD may include one or more selected from the group consisting of derivatives of CBD, stereoisomers thereof, pharmaceutically acceptable salts thereof, hydrates thereof, and solvates thereof.

As used herein, the term "pharmaceutically acceptable salt" refers to a salt in a pharmaceutically usable form of a substance in which cations and anions are bound by electrostatic attraction, which can typically be a metal salt, a salt with an organic base, a salt with an inorganic acid, a salt with an organic acid, a salt with a basic or acidic amino acid, etc. For example, a metal salt can be an alkali metal salt (sodium salt, potassium salt, etc.), an alkali earth metal salt (calcium salt, magnesium salt, barium salt, etc.), an aluminum salt, etc., salts with organic bases can be salts with triethylamine, pyridine, picoline, 2,6-lutidine, ethanolamine, diethanolamine, triethanolamine, cyclohexylamine, dicyclohexylamine, N,N-dibenzylethylenediamine, etc., salts with inorganic acids can be salts with hydrochloric acid, hydrobromic acid, nitric acid, sulfuric acid, phosphoric acid, etc., salts with organic acids, which may include salts with formic acid, acetic acid, trifluoroacetic acid, phthalic acid, fumaric acid, oxalic acid, tartaric acid, maleic acid, citric acid, succinic acid, methanesulfonic acid, benzenesulfonic acid, p-toluenesulfonic acid, etc., salts with basic amino acids can be salts with arginine, lysine, ornithine etc., salts with acidic amino acids can be salts with aspartic acid, glutamic acid etc. Particularly desirable salts are, when the compound has an acidic functional group therein, inorganic salts such as alkali metal salts (for example, sodium salts, potassium salts, etc.), alkali earth metal salts (for example, calcium salts, magnesium salts, barium salts, etc.), and organic salts such as ammonium salts, and when the compound has a basic functional group therein, particularly desirable salts are salts with inorganic acids such as hydrochloric acid, hydrofluoric acid, nitric acid, sulfuric acid, phosphoric acid, etc., and salts with organic acids such as acetic acid, phthalic acid, fumaric acid, oxalic acid, tartaric acid, maleic acid, citric acid, succinic acid, methanesulfonic acid, p-toluenesulfonic acid, etc.

As used herein, the term "cancer" refers to an abnormal growth, or disease that forms a tumor, caused by the autonomous overgrowth of body tissue. The cancer may be a solid cancer, and more specifically may be lung cancer. In addition, the term "lung cancer" refers to a malignant tumor in the lungs, which can be broadly divided into primary lung cancer (occurring in the lung position) and metastatic lung cancer. The type of primary lung cancer is divided into non-small cell lung cancer and small cell lung cancer based on the size and shape of cancer cells, and most of them are non-small cell lung cancer, which is divided into adenocarcinoma, squamous cell carcinoma, and large cell carcinoma.

In the compositions, the CBD and the anti-cancer agent may be co-administered, specifically, the CBD may be administered simultaneously in one formulation with the anti-cancer agent, or may be administered simultaneously or sequentially in separate formulations with the anti-cancer agent.

As used herein, the term "co-administration" may refer to the simultaneous, sequential, or separate administration of the individual components of a treatment regimen. Combination therapy may be defined as achieving a combined therapeutic effect by administering two or more drugs simultaneously or sequentially, or alternately at regular or undetermined intervals, such that the efficacy, as measured, for example, by degree of response, rate of response, duration of disease progression, or survival period, is therapeutically superior to the efficacy achieved by administering one or the other of the components of the combination therapy at usual doses and may provide synergistic effects.

The composition may include the CBD and the anti-cancer agent in a concentration ratio of 9:1 to 1:9. Specifically, the concentration ratio of CBD and anti-cancer agent (CBD:anti-cancer agent) may be 9:1 to 1:9, 9:1 to 1:7, 9:1 to 1:5, 9:1 to 1:4, 9:1 to 1:3, 9:1 to 1:2, 9:1 to 1:1, 7:1 to 1: 9, 7:1 to 1:7, 7:1 to 1:5, 7:1 to 1:4, 7:1 to 1:3, 7:1 to 1:2, 7:1 to 1:1, 5:1 to 1:9, 5:1 to 1:7, 5:1 to 1:5, 5:1 to 1:4, 5:1 to 1:3, 5:1 to 1:2, 5:1 to 1:1, 4:1 to 1: 9, 4:1 to 1:7, 4:1 to 1:5, 4:1 to 1:4, 4:1 to 1:3, 4:1 to 1:2, 4:1 to 1:1, 3:1 to 1:9, 3:1 to 1:7, 3:1 to 1:5, 3:1 to 1:4, 3:1 to 1:3, 3:1 to 1:2, 3:1 to 1:1, 2:1 to 1: 9, 2:1 to 1:7, 2:1 to 1:5, 2:1 to 1:4, 2:1 to 1:3, 2:1 to 1:2, 2:1 to 1:1, 1:1 to 1:9, 1:1 to 1:7, 1:1 to 1:5, 1:1 to 1:4, 1:1 to 1:3, or 1:1 to 1:2.

The CBD may be included in the composition at a concentration of 1 to 50 µM. Specifically, the CBD may be included in the composition at a concentration of 1 to 50 µM, 1 to 45 µM, 1 to 40 µM, 1 to 35 µM, 1 to 30 µM, 1 to 25 µM, 1 to 20 µM, 1 to 15 µM, 5 to 50 µM, 5 to 45 µM, 5 to 40 µM, 5 to 35 µM, 5 to 30 µM, 5 to 25 µM, 5 to 20 µM, 5 to 15 µM, 10 to 50 µM, 10 to 45 µM, 10 to 40 µM, 10 to 35 µM, 10 to 30 µM, 10 to 25 µM, 10 to 20 µM, 10 to 15 µM, 15 to 50 µM, 15 to 45 µM, 15 to 40 µM, 15 to 35 µM, 15 to 30 µM, 15 to 25 µM, 15 to 20 µM, 20 to 50 µM, 20 to 45 µM, 20 to 40 µM, 20 to 35 µM, 20 to 30 µM, or 20 to 25 µM.

In addition, the CBD may be included in the composition at a concentration range that does not exhibit toxic, inhibitory, and/or killing activity against lung cancer cells or lung cancer tissue.

The anti-cancer agent may be included in the composition at a concentration of 1 to 150 µM. Specifically, the anti-cancer agent may be included in the composition at a concentration of 1 to 150 µM, 1 to 130 µM, 1 to 100 µM, 1 to 80 µM, 1 to 60 µM, 1 to 50 µM, 1 to 45 µM, 1 to 40 µM, 1 to 35 µM, 1 to 30 µM, 1 to 25 µM, 1 to 20 µM, 1 to 15 µM, 5 to 150 µM, 5 to 130 µM, 5 to 100 µM, 5 to 80 µM, 5 to 60 µM, 5 to 50 µM, 5 to 45 µM, 5 to 40 µM, 5 to 35 µM, 5 to 30 µM, 5 to 25 µM, 5 to 20 µM, 5 to 15 µM, 10 to 150 µM, 10 to 130 µM, 10 to 100 µM, 10 to 80 µM, 10 to 60 µM, 10 to 50 µM, 10 to 45 µM, 10 to 40 µM, 10 to 35 µM, 10 to 30 µM, 10 to 25 µM, 10 to 20 µM, 10 to 15 µM, 15 to 150 µM, 15 to 130 µM, 15 to 100 µM, 15 to 80 µM, 15 to 60 µM, 15 to 50 µM, 15 to 45 µM, 15 to 40 µM, 15 to 35 µM, 15 to 30 µM, 15 to 25 µM, 15 to 20 µM, 20 to 150 µM, 20 to 130 µM, 20 to 100 µM, 20 to 80 µM, 20 to 60 µM, 20 to 50 µM, 20 to 45 µM, 20 to 40 µM, 20 to 35 µM, 20 to 30 µM, or 20 to 25 µM.

As used herein, the term "etoposide" refers to the compound represented by Formula 2 below, which is a chemotherapeutic anti-cancer agent used to treat several types of cancer, including testicular cancer, lung cancer, lymphoma, leukemia, neuroblastoma, and ovarian cancer.

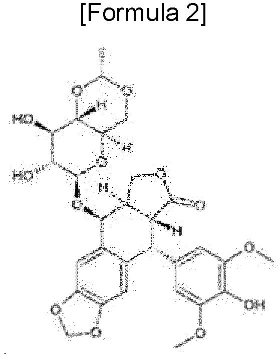

The etoposide may include one or more selected from the group consisting of derivatives of etoposide, stereoisomers thereof, pharmaceutically acceptable salts thereof, hydrates thereof, and solvates thereof.

The term "treatment" used herein refers to any act of ameliorating or beneficially modifying the symptoms of a lung cancer disease by administration of a composition of the disclosure.

As used herein, the term "prevent" refers to any act by which the development of a lung cancer disease or condition is inhibited or delayed by administration of a composition of the disclosure.

The compositions may more effectively inhibit proliferation and induce apoptosis of lung cancer cells by co-administering CBD and the anti-cancer agent. Specifically, the composition may induce or enhance one or more of the effects of apoptosis and autophagy in lung cancer cells.

According to one embodiment, it was demonstrated that the composition induces increased expression of PARP [Poly (ADP-ribose) polymerase], cleaved caspase9 and cleaved caspase8 proteins, which are apoptosis-related factors, and increased expression of LC3-II protein, which is an autophagy-related factor, and may be observed that the co-administration of CBD and the anti-cancer agent significantly enhances the anti-cancer effect.

The CBD may enhance the anticancer effect of the anticancer drug against lung cancer, and specifically, may enhance the anticancer effect of Etoposide on lung cancer. The CBD may improve or enhance the cancer therapeutic effect of the anti-cancer agent by inducing or enhancing the activity of the anti-cancer agent, reducing the resistance of cancer cells to the anti-cancer agent, or increasing the sensitivity of cancer cells to the anti-cancer agent.

The pharmaceutical composition may include a pharmaceutically acceptable carrier. The term "pharmaceutically acceptable carrier" may refer to a carrier or diluent that does not irritate the organism and does not interfere with the biological activity and properties of the compound being administered. In this context, "pharmaceutically acceptable" refers to not inhibiting the activity of the active ingredient and does not have more than adaptable toxicity to the subject to whom it is applied (prescribed). The type of carrier that may be used in the pharmaceutical composition may be any carrier commonly used and pharmaceutically acceptable in the art. Non-limiting examples of the carriers include lactose, dextrose, maltodextrin, sucrose, sorbitol, mannitol, xylitol, erythritol, maltitol, glycerol, ethanol, starch, acacia gum, alginate, gelatin, calcium phosphate, calcium silicate, cellulose, methyl cellulose, microcrystalline cellulose, polyvinyl pyrrolidone, water, saline, sterile water, Ringer's solution, buffered saline, albumin injection solution, methylhydroxybenzoate, propylhydroxybenzoate, talc, magnesium stearate, or mineral oil, etc. These may be used either individually or in combination with two or more types. The pharmaceutical composition may be formulated as an oral dosage form or a parenteral formulation, depending on the route of administration, by common methods known in the art, including pharmaceutically acceptable carriers in addition to the active ingredient.

The pharmaceutical compositions may be formulated and used according to common methods in the form of oral dosage forms, topical preparations, suppositories, or sterile injectable solutions, such as pills, granules, tablets, capsules, suspensions, emulsions, syrups, aerosols, etc. The pharmaceutical compositions may be formulated by adding diluents or excipients such as commonly used fillers, extenders, binders, lubricants, disintegrants, or surfactants, etc.

When the pharmaceutical composition is formulated as an oral dosage form, the pharmaceutical composition may be prepared as a powder, granule, tablet, pill, disintegrating tablet, capsule, liquid, gel, syrup, suspension, wafer, etc., in combination with a suitable carrier, according to methods known in the art. Examples of suitable pharmaceutically acceptable carriers include sugars such as lactose, glucose, sucrose, dextrose, sorbitol, mannitol, and xylitol, etc., starches such as corn starch, potato starch, and wheat starch, etc., celluloses such as cellulose, methylcellulose, and ethylcellulose, sodium carboxymethylcellulose and hydroxypropylmethylcellulose, etc., polyvinyl pyrrolidone, water, methylhydroxybenzoate, propylhydroxybenzoate, magnesium stearate, mineral oils, malt, gelatin, talc, polyols, vegetable oils, etc. In the case of formulation, if necessary, the pharmaceutical composition may be formulated to include diluents and/or excipients such as fillers, extenders, binders, lubricants, disintegrants, surfactants, etc.

When the pharmaceutical composition is formulated as a parenteral formulation, the pharmaceutical composition may be formulated in the form of an injectable formulation, transdermal delivery system, nasal inhaler, and suppository according to methods known in the art in combination with a suitable carrier. When formulated as an injectable formulation, sterile water, ethanol, polyols such as glycerol or propylene glycol, etc., or mixtures thereof, may be used as suitable carriers, desirably isotonic solutions such as Ringer's solution, phosphate buffered saline (PBS) containing triethanolamine, sterile injectable water, 5% dextrose, etc., may be used. When formulated as a transdermal delivery system, the pharmaceutical composition may be formulated as an ointment, a cream, a lotion, a gel, a topical solution, a paste, a liniment, or an aerosol, etc. In the case of nasal inhalers, the pharmaceutical composition may be formulated as an aerosol spray using a suitable propellant such as dichlorofluoromethane, trichlorofluoromethane, dichlorotetrafluoroethane, carbon dioxide, etc., when formulated as a suppository, witepsol, tween 61, polyethylene glycol, cacao glycol, laurin glycol, polyoxyethylene sorbitan fatty acid ester, polyoxyethylene stearate, sorbitan fatty acid ester, etc., may be used as the base.

The pharmaceutical composition may be administered in a pharmaceutically effective amount, wherein the term "pharmaceutically effective amount" refers to an amount sufficient to treat or prevent a disease with a reasonable benefit/risk ratio applicable to medical treatment or prevention, and an effective dose level may be determined by factors including severity of the disease, activity of a drug, age, weight, health, and sex of a patient, sensitivity of the patient to a drug, time of administration, route of administration, and elimination rate of the composition of the disclosure used, duration of treatment, the drugs used in combination or concurrently with the composition of the disclosure, and other factors well known in the medical field. The pharmaceutical composition may be administered alone or in combination with components known to exhibit therapeutic effects against lung cancer. It is important to administer an amount that can obtain the maximum effect with the minimum amount without side effects in consideration of all the above factors.

The dosage of the pharmaceutical composition can be determined by those skilled in the art, taking into account the intended use, the severity of the disease, the patient's age, weight, gender, pre-existing conditions, or the type of substance used as an active ingredient, etc. For example, the pharmaceutical compositions of the disclosure can be administered at a dose of from 0.1 ng to 1,000 mg/kg per adult, desirably from 1 ng to 100 mg/kg, and the frequency of administration of the compositions of the disclosure is not particularly limited, but can be once daily or multiple times in divided doses. The above dosage or frequency of administration is not intended to limit the scope of the present disclosure in any way.

The pharmaceutical composition for use according to the invention, which comprises CBD and etoposide, enhances anti-cancer effects to lung cancer of etoposide alone.

As used herein, the term "enhances cancer treatment effectiveness" or "enhances anti-cancer effects" may refer to improving and/or enhancing the cancer therapeutic effect of the anti-cancer agent by inducing or enhancing the activity of the anti-cancer agent, reducing the resistance of cancer cells to the anti-cancer agent, or increasing the sensitivity of cancer cells to the anti-cancer agent.

As used herein, the term "subject" may include, without limitation, a mammal, including mice, livestock, humans, etc., a bird, a reptile, and farmed fish, etc., in which the subject has or is at risk of developing lung cancer disease, and the term "subject" may exclude humans.

The pharmaceutical composition may be administered in a single or multiple doses in a pharmaceutically effective amount. The composition may be formulated and administered in the form of a liquid, an acid, an aerosol, an injectable formulation, an aqueous solution (ringel), a capsule, a pill, a tablet, a suppository, or a patch. The route of administration of the pharmaceutical composition for the prevention or treatment of lung cancer may be by any conventional route as long as the pharmaceutical composition can reach the target tissue.

The pharmaceutical composition may be administered by any of the following routes, depending on the intended use, intraperitoneal administration, intravenous administration, intramuscular administration, subcutaneous administration, intradermal administration, transdermal patch administration, oral administration, intranasal administration, intrapulmonary administration, or rectal administration, etc. However, since oral administration may be in an unformulated form, and since stomach acid may denature or degrade the active ingredient of the pharmaceutical composition, the oral composition may be formulated to coat the active agent or to protect it from degradation in the stomach, or may be administered orally in the form of an oral patch. In addition, the composition may be administered by any device that allows the active agent to be transported to the target cell.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a diagram illustrating the cannabidiol (CBD) manufacturing process;
FIG. 2 is a diagram illustrating the survival rate of a A549 cell line in response to treatment with different concentrations of cannabidiol (CBD);
FIG. 3 is a diagram illustrating the survival rate of the A549 cell line in response to combined treatment with CBD and etoposide;
FIG. 4 is a diagram illustrating microscopic observations of the A549 cell line status in response to combined treatment with CBD and etoposide;
FIG. 5 is a diagram illustrating the results of western blotting analysis of the expression levels of cleaved PARP[Poly (ADP-ribose) polymerase], cleaved caspase9, cleaved caspase8, Bax, Bcl-2, and LC3B in the A549 cell line in response to combined treatment with CBD and etoposide; and
FIG. 6A and 6B are diagrams confirming the results of apoptosis of the A549 cell line in response to combined treatment with CBD and etoposide.

### DETAILED DESCRIPTION

Examples of the pharmaceutical composition for use according to the invention are illustrated in the accompanying drawings, wherein like reference numerals refer to like elements throughout. The examples are merely described below, by referring to the figures, to explain aspects of the present invention. As used herein, the term "and/or" includes any and all combinations of one or more of the associated listed items. Expressions such as "at least one of," when preceding a list of elements, modify the entire list of elements and do not modify the individual elements of the list.

This will be explained in more detail in the following examples. However, these examples are for illustrative purposes only and the scope of the invention is not limited to these examples.

In addition, the cannabidiol (CBD) compounds used in the following embodiments were isolated from *Cannabis* by NeoKenBio, Inc. under the narcotic drug (*Cannabis*) academic researcher license (No. 1564, 1979, and 2083) of the Seoul Regional Food and Drug Safety Administration.

### Example 1: Manufacturing process of cannabidiol (CBD)

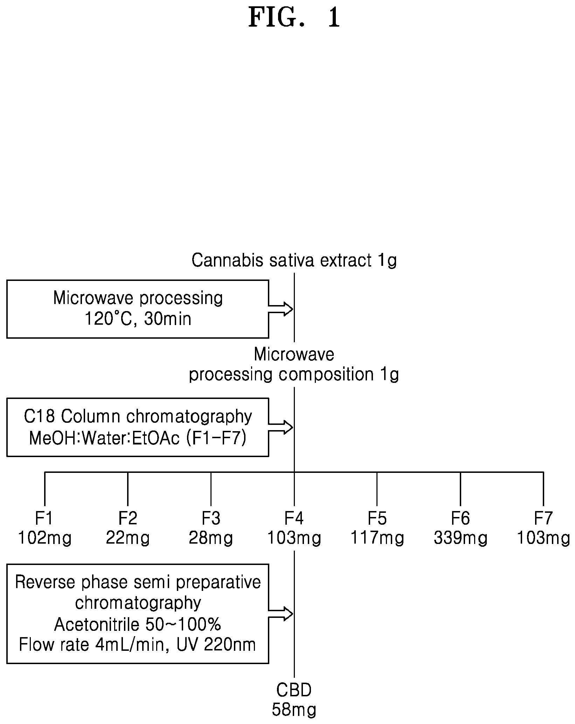

The CBD compound was prepared and obtained by the following method (Fig 1).

First, 200 g of chopped dry *Cannabis* (*Cannabis* leaves or *Cannabis* flowers) were subjected to ultrasonic extraction with 2 L of ethyl acetate for 1 hour, followed by extraction at room temperature for 24 hours, and the process was repeated twice. The extract was concentrated by evaporation under reduced pressure to obtain 17.6 g of a dry extract containing cannabidiolic acid (CBDA) and CBD.

Next, the *Cannabis* extract obtained above was subjected to microwave processing. Specifically, 1 g of the *Cannabis* extract was added to 2 mL of ethyl acetate in a container of a microwave irradiator (model number 908005) manufactured by CEM, USA, and processed at 100 W, frequency 2450 MHz at 120°C for 30 minutes after closing the container stopper, and 1 g of the processed composition was prepared by evaporative concentration under reduced pressure.

Next, 1 g of the microwave processed composition was adsorbed on 2 g of C18 (Nacalai tesque, Cosmosil C18), and then a glass column with an inner diameter of 2.8 cm was filled with C18 to a height of 10.0 cm and separated by flowing a solvent mixed with methanol and water and ethyl acetate. The eluting solvents were 50 %, 60 %, 70 %, 80 %, 90 %, and 100 % methanol and 100 % ethyl acetate, resulting in seven fractions labeled F1 to F7. Each fraction yielded 102 mg (fraction F1), 22 mg (fraction F2), 28 mg (fraction F3), 103 mg (fraction F4), 117 mg (fraction F5), 339 mg (fraction F6), and 103 mg (fraction F7). No CBD was identified in fraction F1 to fraction F3 and fraction F5 to fraction F7, but fraction F4 was found to contain a large amount of CBD.

Next, separation of CBD from the F4 fraction obtained above was attempted. Specifically, by reverse-phase semi preparative chromatography (stationary phase: Luna C18 (2) column from Phenomenex, particle size 10 µm, length 250 mm x 10 mm), the eluate was developed with an initial acetonitrile:water=50:50 (v/v) and increased to acetonitrile:water=100:0 (v/v) at a flow rate of 4 mL/min for 60 minutes to 90 minutes to obtain one major peak CBD appearing at UV 220 nm. The CBD is over 99.8 % pure and the amount isolated is 58 mg.

### Example 2: Evaluation of cancer cell toxicity of cannabidiol (CBD)

To evaluate the toxicity of CBD to lung cancer cells, the following experiments were conducted.

Specifically, A549 cells, a lung cancer cell line, were seeded in 96-well plates at a concentration of 1 X 10⁴ cells/well, cultured in a 37 °C, 10 % CO₂ incubator for 24 hours, then treated with CBD in a concentration-dependent manner and cultured under the same conditions for 48 hours. After completion of culture, 1/10 of WST-8 solution (Quanti-MaxTM, BIOMAX, Korea) was added to the medium, and after additional culture at 37 °C for 1 hour, the cell survival rate was determined by measuring absorbance at 450 nm, and expressed as a percentage of the control without any treatment. The experiment was conducted independently and repeated three times.

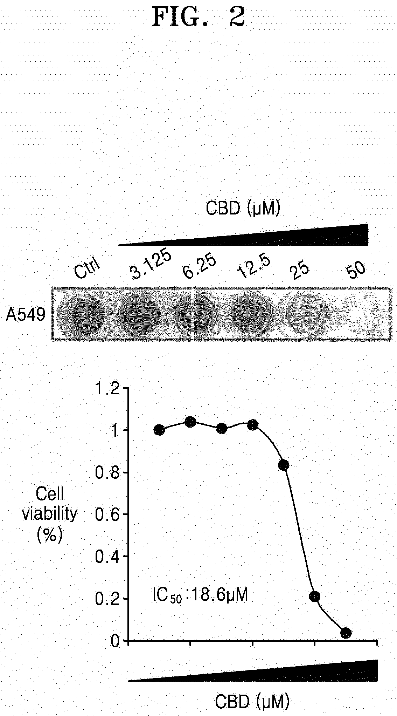

As a result, the IC₅₀ value for CBD in A549 cells was determined to be 18.6 µM (FIG. 2), indicating that below this concentration, CBD does not exhibit toxicity to A549 cells. In subsequent experiments, the concentration of CBD was set to 15 µM.

### Example 3: Identify anti-cancer agents with enhanced anti-cancer efficacy by cannabidiol (CBD)

To identify anti-cancer agents whose anti-cancer efficacy can be enhanced by CBD, the following experiments were conducted.

Specifically, A549 cells, a lung cancer cell line, were seeded in 96-well plates at a concentration of 1×10⁴ cells/well, cultured for 24 hours in a 37 °C, 10 % CO₂ incubator, and then treated with anti-cancer agents not currently used in non-small cell lung cancer in the presence or absence of CBD (15 µM) in a concentration-dependent manner, and cultured under the same conditions for 48 hours. After completion of culture, 1/10 of WST-8 solution (Quanti-MaxTM, BIOMAX, Korea) was added to the medium, and after additional culture at 37 °C for 1 hour, the cell survival rate was determined by measuring absorbance at 450 nm, and expressed as a percentage of the control without any treatment. The experiment was conducted independently and repeated three times.

As a result, based on the IC₅₀ for the combination treatment of anticancer drugs and CBD in A549 cells, it was confirmed that CBD can enhance the cancer cytotoxic effect of several anticancer drugs, and in particular, it was confirmed that the cancer cytotoxic effect was significantly enhanced when combination treated with etoposide compared to other anti-cancer agents (Table 1). Therefore, in the context of lung cancer (specifically non-small cell lung cancer), CBD can significantly increase the anti-cancer effect of etoposide among various anti-cancer agents.

**[Table 1]**

| Drug | IC₅₀ (µM), 48 h | |
|---|---|---|
| | without CBD | with CBD |
| Etoposide (VP-16) | N/D | 51.43 |
| Tamoxifen | 23.94 | 16.74 |
| Sorafenib | 8.97 | 4.26 |
| Cabozantinib (XL184) | 8.29 | 4.14 |
| Lenvatinib | 19.86 | 16.95 |
| Regorafenib | 5.27 | 4.11 |
| Carboplatin | N/D | > 100 |
| Vinorelbine | 12.40 | 6.38 |

### Example 4: Evaluation of cancer cell killing efficacy of cannabidiol (CBD) and etoposide combined treatment

To evaluate the cancer cell killing efficacy of etoposide, a known lung cancer therapeutic agent, in combined treatment with CBD, the following experiments were conducted.

Specifically, A549 cells, a lung cancer cell line, were seeded in 96-well plates at a concentration of 1 X 10⁴ cells/well, cultured in a 37 °C, 10 % CO₂ incubator for 24 hours, then treated with etoposide by concentration, treated with or without CBD (15 µM), and cultured under the same conditions for 48 hours. After completion of culture, 1/10 of WST-8 solution (Quanti-MaxTM, BIOMAX, Korea) was added to the medium, and after additional culture at 37 °C for 1 hour, the cell survival rate was determined by measuring absorbance at 450 nm, and expressed as a percentage of the control without any treatment. The experiment was conducted independently and repeated three times.

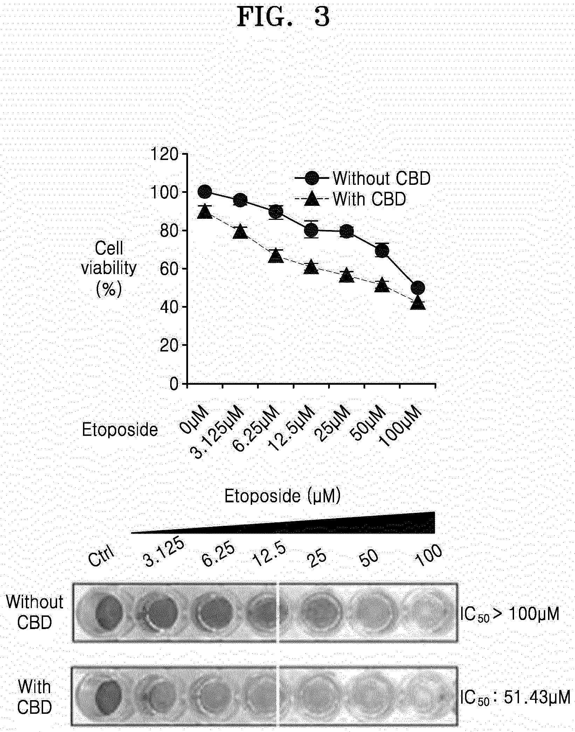

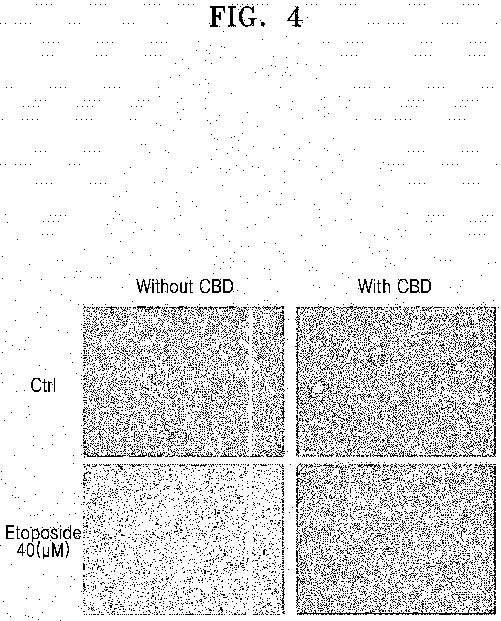

The results confirmed that the toxicity of etoposide to A549 cells was significantly increased by combined treatment with CBD and etoposide compared to treating etoposide alone, and specifically, the cytotoxicity (IC₅₀) against etoposide was found to be increased by about 50 % from 106.4 µM to 51.43 µM upon combined treatment with CBD (FIG. 3 and FIG. 4).

Therefore, based on the above results, it can be seen that combination treatment of etoposide and CBD can significantly increase the anti-cancer effect of etoposide against lung cancer.

### Example 5: Mechanism of action analysis of anti-cancer effect of combined treatment with cannabidiol (CBD) and etoposide

To analyze the mechanism of action of the lung cancer cell killing effect in response to the combined treatment of etoposide and CBD, the following experiments were conducted.

Specifically, A549 cells, a lung cancer cell line, were seeded in 6-well plates at a concentration of 3 X 10⁵ cells/well, cultured in a 37 °C, 10 % CO₂ incubator for 24 hours, then treated with etoposide by concentration, treated with or without CBD (15 µM), and cultured under the same conditions for 24 hours. After completion of culture, the cells were harvested and the proteins were quantified using the Bradford assay (Bio-Rad, Japan), and the proteins (30 ug/well) were separated by electrophoresis on NuPAGE 4-12% Bis-Tris gels (Invitrogen) and transferred to polyvinylidene difluoride (PVDF). Next, the PVDF membrane was reacted with a primary antibody (Monoclonal antibody, Cell signaling technology, USA) specific for PARP[Poly (ADP-ribose) polymerase], cleaved caspase9, cleaved caspase8, LC3B or GAPDH for 1 hour at room temperature, followed by reacting with an anti-rabbit secondary antibody (Cell signaling technology) for 1 hour. The antibodies bound to PVDF were visualized using ECL Advance Western Blotting Detection Reagents (GE Healthcare, UK) and LAS 4000 imaging system (Fujifilm, Japan).

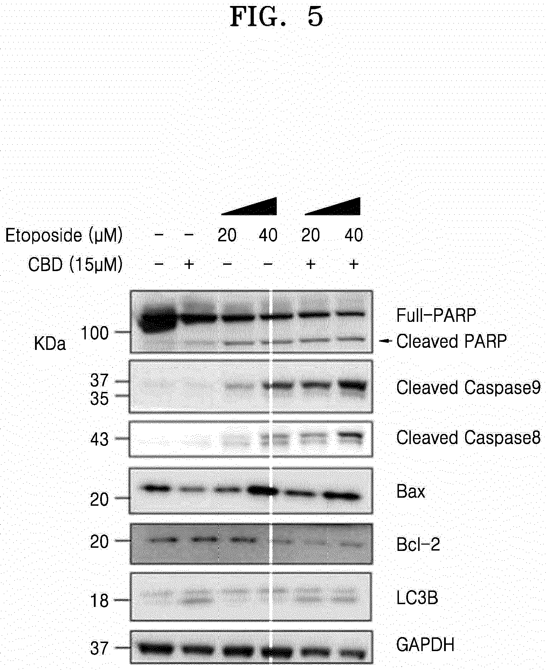

The results confirmed that combined treatment with etoposide and CBD increased the expression of apoptosis-related factors cleaved PARP, cleaved caspase9, cleaved caspase8 and Bax proteins in an etoposide concentration-dependent manner, and conversely, decreased the expression of Bcl-2 protein, which inhibits apoptosis, was observed. Additionally, it was confirmed that the expression of the autophagy-related protein LC3-II also increased (Fig. 5).

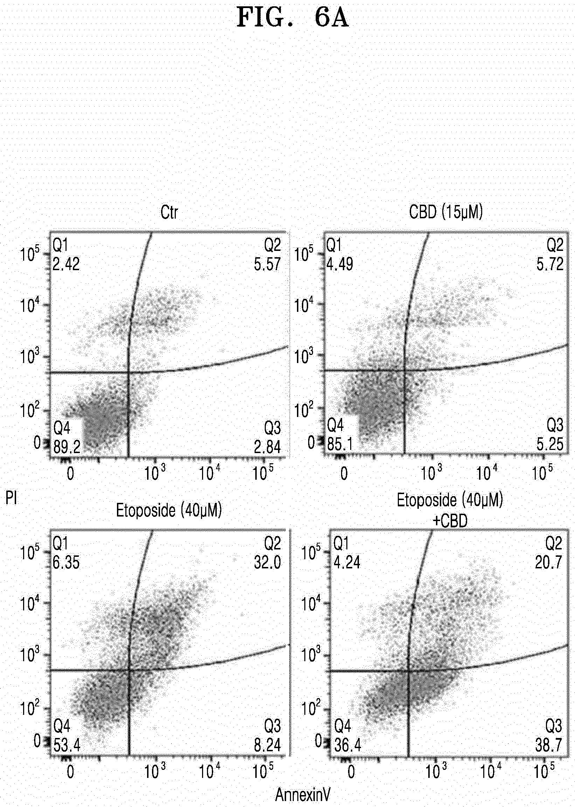

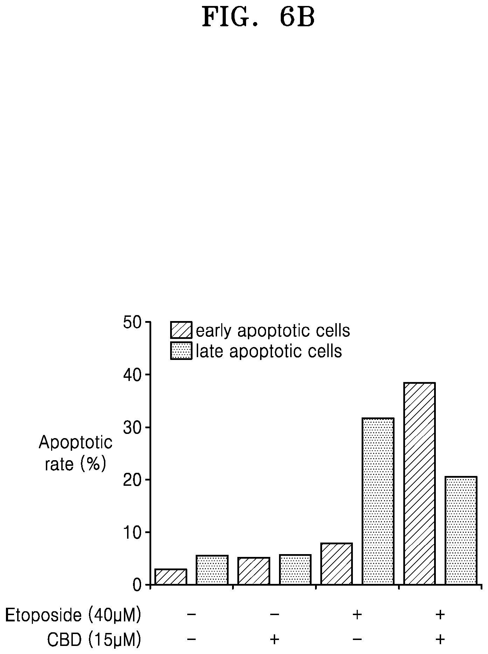

In addition, the apoptosis results of the combination treatment were re-validated using a Fluorescence Activated Cell Sorting (FACS) system. Ultimately, it was confirmed that the number of early apoptotic cells significantly increased in the combined treatment compared to the individual treatment (FIG. 6).

Based on the above results, it can be seen that the combined treatment of etoposide and CBD can induce cancer cell death by inducing apoptosis or autophagy of cancer cells.

The compositions of the invention have been confirmed to exhibit significantly superior lung cancer inhibitory activity when co-treated with CBD and etoposide, one of the anti-cancer agents for lung cancer, and the compositions can be utilized to enhance the therapeutic effect of lung cancer.

## Claims

1. A pharmaceutical composition for use in preventing or treating lung cancer, comprising cannabidiol (CBD) and an anti-cancer agent as active ingredients,
wherein the anti-cancer agent is etoposide.

2. The pharmaceutical composition for use of claim 1, wherein the CBD and anti-cancer agent are co-administered.

3. The pharmaceutical composition for use of claim 1, wherein the CBD and the anti-cancer agent are administered simultaneously in one formulation, or in separate formulations either simultaneously or sequentially.

4. The pharmaceutical composition for use of claim 1, wherein the CBD and the anti-cancer agent are comprised in a concentration ratio of about 9:1 to about 1:9.

5. The pharmaceutical composition for use of claim 1, wherein the CBD comprises one or more selected from the group consisting of pharmaceutically acceptable salts of CBD, hydrates thereof, and solvates thereof.

## Patentansprüche

1. Pharmazeutische Zusammensetzung zur Verwendung bei der Vorbeugung oder Behandlung von Lungenkrebs, umfassend Cannabidiol (CBD) und ein Antikrebsmittel als Wirkstoffe,
wobei das Antikrebsmittel Etoposid ist.

2. Pharmazeutische Zusammensetzung zur Verwendung nach Anspruch 1, wobei das CBD und das Antikrebsmittel gemeinsam verabreicht werden.

3. Pharmazeutische Zusammensetzung zur Verwendung nach Anspruch 1, wobei das CBD und das Antikrebsmittel gleichzeitig in einer einzigen Formulierung oder in getrennten Formulierungen entweder gleichzeitig oder nacheinander verabreicht werden.

4. Pharmazeutische Zusammensetzung zur Verwendung nach Anspruch 1, wobei das CBD und das Antikrebsmittel in einem Konzentrationsverhältnis von etwa 9:1 bis etwa 1:9 enthalten sind.

5. Pharmazeutische Zusammensetzung zur Verwendung nach Anspruch 1, wobei das CBD ein oder mehrere Elemente umfasst, die aus der Gruppe bestehend aus pharmakologisch verträglichen Salzen des CBD, deren Hydraten und deren Solvaten ausgewählt werden.

## Revendications

1. Composition pharmaceutique pour son utilisation dans la prévention ou le traitement du cancer du poumon, comprenant du cannabidiol (CBD) et un agent anticancéreux comme principes actifs,
dans laquelle l'agent anticancéreux est l'étoposide.

2. La composition pharmaceutique pour son utilisation selon la revendication 1, dans laquelle le CBD et l'agent anticancéreux sont coadministrés.

3. La composition pharmaceutique pour son utilisation selon la revendication 1, dans laquelle le CBD et l'agent anticancéreux sont administrés simultanément dans une seule formulation, ou dans des formulations séparées soit simultanément soit séquentiellement.

4. La composition pharmaceutique pour son utilisation selon la revendication 1, dans laquelle le CBD et l'agent anticancéreux sont présents dans un rapport de concentration d'environ 9:1 à environ 1:9.

5. La composition pharmaceutique pour son utilisation selon la revendication 1, dans laquelle le CBD comprend un ou plusieurs éléments choisis dans le groupe constitué par les sels pharmaceutiquement acceptables de CBD, leurs hydrates, et leurs solvates.
